# EUROPÄISCHE PATENTSCHRIFT

(11) **EP 0 447 865 B1**
(45) Veröffentlichungstag und Bekanntmachung des Hinweises auf die Patenterteilung: **03.05.1995**
(21) Anmeldenummer: 91103264.7
(22) Anmeldetag: 05.03.1991
(51) Int. Cl.: A61B 17/22

(54) **Vorrichtung zur Stosswellenbehandlung**
Device for shock waves treatment
Dispositif de traitement pour ondes de choc

(30) Priorität: 10.03.1990 DE 4007669
(43) Veröffentlichungstag der Anmeldung: 25.09.1991
(73) Patentinhaber: Richard Wolf GmbH, 75438 Knittlingen (DE)
(72) Erfinder: Kraus, Werner, W-7134 Knittlingen (DE); Wurster, Helmut, W-7519 Oberderdingen (DE); Belikan, Thomas, W-7134 Knittlingen (DE); Fladl, Joachim, W-7519 Oberderdingen 3 (DE)
(74) Vertreter: Vollmann, Heiko, Dipl.-Ing.

(56) Entgegenhaltungen:
- EP-A- 0 131 654
- EP-A- 0 227 929
- EP-A- 0 423 534
- DE-A- 3 503 702
- DE-C- 3 532 678
- US-A- 4 798 196

## Beschreibung

Die Erfindung geht aus von einer Vorrichtung zur Stoßwellenbehandlung, wie sie aus der nachveröffentlichten, zum Stand der Technik gemäß Artikel 54(3) EPÜ gehörenden Druckschrift EP-A1-0 423 534 bekannt ist. Dieselbe weist zwei Ortungseinrichtungen, und zwar eine Ultraschallortungseinrichtung und eine Röntgenortungseinrichtung auf, die die Kalotte des Stoßwellenwandlers durchdringen und in die Vorlaufstrecke desselben hineinragen. Die Röntgenortungseinrichtung weist einen innerhalb der Vorlaufstrecke angeordneten Tubus auf, der an seinem zur Membran gerichteten Ende mit einem Ballon abgeschlossen ist. Tubus und Ballon sind gegenüber dem Ankoppelmedium der Vorlaufstrecke abgedichtet und mit einem Gas z. B. Luft füllbar, um die zur Ortung dienenden Röntgenstrahlen möglichst wenig zu dämpfen. Beim Betrieb der Vorrichtung werden Tubus und Ballon derart mit Gas gefüllt, daß sich der Ballon an die Innenseite der Membran und somit mittelbar an den Körper der zu behandelnden Person anlegt, wodurch erreicht wird, daß das flüssige innerhalb der Vorlaufstrecke befindliche, die Röntgenstrahlung stark dämpfende Koppelmedium fast vollständig aus dem Röntgenstrahlengang verdrängt wird.

Durch diese Maßnahme wird die Röntgenortung im erheblichen Maße verbessert, da die die Gasstrecke durchlaufenden Röntgenstrahlen eine wesentlich geringere Dämpfung erfahren, als dies bei einem flüssigen Ankoppelmedium der Fall wäre. Als Nachteil hat es sich jedoch erwiesen, daß der Tubus aufgrund seiner für eine gute Röntgenortung nötigen Länge einen relativ großen Teil des Schallfeldes des Stoßwellenwandlers abschattet. Eine solche Abschattung tritt auch durch den Ballon auf, so daß eine Verkürzung des Tubus bei entsprechender Vergrößerung des Ballons zumindest unter dem Aspekt der Abschattung keine Verbesserung darstellt, denn der Ballon muß zur Röntgenortung soweit aufgedehnt werden, daß er sich nicht mehr dem Röntgenkegel anpassen kann und einen relativ großen Teil des Schallfeldes abschattet. Es hat sich gezeigt, daß eine solche Abschattung des Schallfeldes die Effizienz der Stoßwellentherapie beeinträchtigen kann.

Hiervon ausgehend liegt der Erfindung die Aufgabe zugrunde, insbesondere die Röntgenortungseinrichtung bei der bekannten Vorrichtung so auszubilden, daß sowohl die Stoßwellentherapie als auch die Röntgenortung ohne gegenseitige störende Beeinflussung unter optimalen Bedingungen durchgeführt werden können.

Diese Aufgabe wird dadurch gelöst, daß der Tubus innerhalb der Vorlaufstrecke in seiner axialen Richtung längenverstellbar ausgebildet ist. Hierdurch ist es bei der Stoßwellentherapie möglich, der gestellten Anforderung entsprechend den Tubus hinsichtlich seiner Länge soweit zu reduzieren, daß das Schallfeld des Stoßwellenwandlers nicht oder nur geringfügig eingeschränkt wird. Die Verkürzung des Röntgentubus ermöglicht zwar die weitere Anwendung der Röntgenortungseinrichtung jedoch mit der Einschränkung, daß aufgrund des innerhalb des Röntgenstrahlenganges befindlichen flüssigen Ankoppelmediums die Auflösung des Röntgenbildes verringert wird. Ist eine höhere Auflösung erforderlich, so muß die Länge des Tubus zur Verdrängung des flüssigen Koppelmediums in Richtung auf die Membran vergrößert werden. Die Erfindung ermöglicht durch die Längenänderung des Tubus sowohl die Röntgenortung als auch die Stoßwellentherapie in optimaler Weise zu betreiben.

Weitere vorteilhafte Ausgestaltungen der Erfindung sind durch die in den Unteransprüchen aufgeführten Merkmale gekennzeichnet.

Vorteilhaft wird die Längenänderung des Tubus durch eine teleskopartige Ausbildung desselben erreicht. Hierdurch kann die Tubuslänge kontinuierlich verändert werden. Die erfindungsgemäße Ausbildung des Tubus weist des weiteren den großen Vorteil auf, daß am Röntgenstrahler selbst keine konstruktiven Veränderungen vorzunehmen sind, wodurch z. B. gattungsgemäße Vorrichtungen älterer Bauart problemlos nachrüstbar sind.

Zweckmäßigerweise besteht der Tubus aus ineinanderschiebbaren konzentrischen Rohrabschnitten, deren Durchmesser entsprechend dem zur Membran hin sich aufweitenden Röntgenstrahl gewählt sind.

In vorteilhafter Weise kann die Länge des Röntgentubus durch Anlegen eines Über- oder Unterdruckes variiert werden. Hierdurch kann einerseits eine stufenlose Längenänderung des Tubus erreicht und zum anderen auf komplizierte mechanische Antriebseinrichtungen im Bereich der Vorlaufstrecke verzichtet werden. Durch die mittels Unter- und Überdruck bewirkte Längenänderung des Röntgentubus in Richtung auf die die Kalotte abschließende Membran ist es zudem unproblematisch, das durch den Tubus in der Vorlaufstrecke verdrängte Ankoppelmedium abzuführen bzw. dieses bei Verkürzung der Tubuslänge wiederum der Vorlaufstrecke zuzuführen. Hierzu kann ein in zwei Kammern unterteilter Ausgleichsbehälter vorgesehen sein, dessen eine Kammer mit der Vorlaufstrecke und dessen andere Kammer mit dem durch eine elastische Membrane oder dergleichen abgeschlossenen Tubusinneren über Leitungen verbunden ist. Ein derartiges Ausgleichssystem ist in der deutschen Patentanmeldung P 39 34 105.4 beschrieben, auf die hier ausdrücklich Bezug genommen wird.

In besonders vorteilhafter Weise ist der erfindungsgemäße Röntgentubus mit einem aufblasbaren Ballon ausgestattet, der innerhalb des Röntgentubus entlang der Tubuswand verläuft und in einfacher Weise,z.B. mittels eines Klemmringes,am kalottennahen Ende des Tubus gasdicht gegen die Tubusinnenwand festgelegt ist. Somit kann die Längenänderung des Tubus über diesen Ballon durch Anlegen eines Über- oder Unterdruckes erfolgen. Durch die Verwendung des Ballons kann auf eine zusätzliche Abdichtung des Tubus gegenüber dem innerhalb der Vorlaufstrecke befindlichen Ankoppelmedium, insbesondere auf eine aufwendige Abdichtung der einzelnen Teleskoprohrabschnitte zueinander, verzichtet werden. Der Ballon kann dabei insbesondere im Bereich der Tubuswand verhältnismäßig dünn ausgebildet sein, da er über seinen gesamten Umfang durch die Tubuswand abgestützt wird.

Um eine Längenänderung des Tubus vornehmen zu können, muß der Ballon neben der Befestigung am kalottennahen Ende des Tubus zusätzlich an dessen freien Ende beispielsweise mittels eines Klemmrings befestigt sein.

Die Erfindung ist nachfolgend anhand eines in den Figuren dargestellten Ausführungsbeispieles näher erläutert, das weitere vorteilhafte Ausgestaltungen der Erfindung verdeutlicht. Es zeigen:
- Figur 1: einen Teil einer in die Kalotte eines Stoßwellenwandlers integrierten Röntgenortungseinrichtung mit einem erfindungsgemäßen Röntgentubus,
- Figur 2: die schematische Darstellung der Kalotte eines Stoßwellenwandlers im Schnitt, mit dem Röntgentubus in der ersten Endstellung,
- Figur 3: die Kalotte des Stoßwellenwandlers nach Figur 2 mit teilweise teleskopisch verlängertem Röntgentubus,
- Figur 4: die Kalotte des Stoßwellenwandlers nach Figur 2 mit dem Röntgentubus in der zweiten Endstellung.

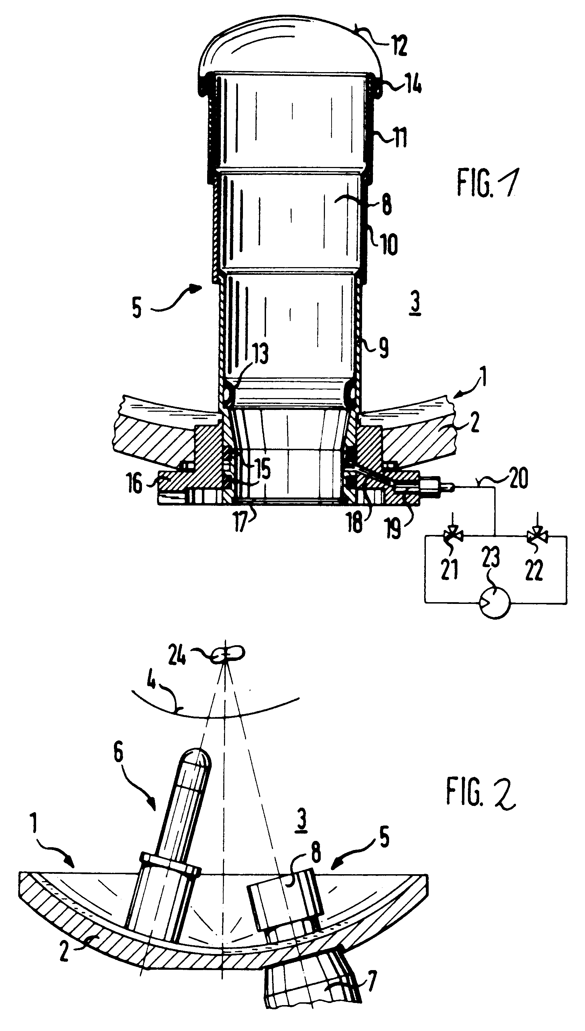

Die in den Figuren nur teilweise dargestellte gattungsgemäße Vorrichtung weist in bekannter Weise einen Stoßwellenwandler mit einer fokussierenden Kalotte 2 mit einer sich daran anschließenden mit einem flüssigen Koppelmedium 3 gefüllten und durch eine in den Figuren 2 bis 4 dargestellte Membran 4 nach außen hin dicht abgeschlossenen Vorlaufstrecke auf.

Zur Durchführung einer Stoßwellenbehandlung ist es erforderlich, das zu behandelnde Konkrement 24 oder Gewebe zu orten und in den Brennpunkt der Kalotte 2 zu bringen. Hierzu sind zwei Ortungseinrichtungen, und zwar eine Röntgenortungseinrichtung 5 und eine Ultraschallortungseinrichtung 6 vorgesehen, welche die Kalotte 2 durchdringen oder in dieser festgelegt sind und in die Vorlaufstrecke hineinragen.

Gegenstand der vorliegenden Erfindung ist die Ausbildung der Röntgenortungseinrichtung 5, weshalb die Ultraschallortungseinrichtung 6 hier nicht im einzelnen beschrieben ist. Es können ggf. weitere Ortungseinrichtungen vorgesehen sein, auch ist die Ultraschallortungseinrichtung 6 selbstverständlich nicht zwingend erforderlich.

Die Röntgenortungseinrichtung 5 weist einen im wesentlichen außerhalb der Kalotte 2 angeordneten Röntgenstrahler 7 auf, dessen längenverstellbarer Tubus 8 in die durch das Koppelmedium 3 ausgefüllte Vorlaufstrecke ragt. Der Röntgentubus 8 besteht in der dargestellten Ausführung aus drei konzentrisch zueinander angeordneten und ineinander einschiebbaren Rohrabschnitten 9, 10 und 11, welche ein Teleskopsystem bilden (siehe Figur 1). Je nach Anforderungen können die Anzahl der Rohrabschnitte und deren Abmessungen größer oder kleiner gewählt werden. Innerhalb des durch die Rohrabschnitte 9, 10 und 11 gebildeten Röntgentubus 8 ist ein elastischer, das Innere des Röntgentubus 8 gegenüber dem Koppelmedium 3 abdichtender Ballon 12 angeordnet. Der Ballon 12 ist an seinem offenen Ende durch einen innerhalb des kalottennahen Rohrabschnitts 9 angeordneten ersten Klemmring 13 fest mit dem Röntgentubus 8 verbunden, wobei der Klemmring 13 den ihn umgebenden Ballon 12 zum gasdichten Abschluß gegen die Innenseite des Rohrabschnitts 9 preßt.

Der den Tubus 8 gas- und flüssigkeitsdicht abschließende Ballon 12 ist kragenförmig über das Ende des Rohrabschnitts 11 gestülpt und durch einen an der Außenseite des Rohrabschnitts 11 angeordneten zweiten Klemmring 14 an diesem festgelegt.

Der Röntgentubus 8 ist mit seinem Rohrabschnitt 9 über Gummidichtungen 15 dicht in einen Sockel 16 eingesetzt, welcher fest und dicht in der Kalotte 2 sitzt. Der Röntgentubus 8 ist an seinem kalottenseitigen Ende durch ein Röntgenfenster 17 dicht gegenüber dem Röntgenstrahler 7 abgeschlossen.

Innerhalb des Sockels 16 und des Rohrabschnitts 9 ist ein Kanal 18 vorgesehen, welcher das Tubusinnere über den Anschluß 19, und die Ventile 21 und 22 mit der Pumpe 23 verbindet.

Die Ventile 21 und 22 können mit den beiden Kammern eines Ausgleichsbehälters nach der deutschen Patentanmeldung P 39 34 105.4 in Verbindung stehen, dessen Kammern einerseits mit Gas und andererseits mit dem flüssigen Koppelmedium 3 gefüllt sind.

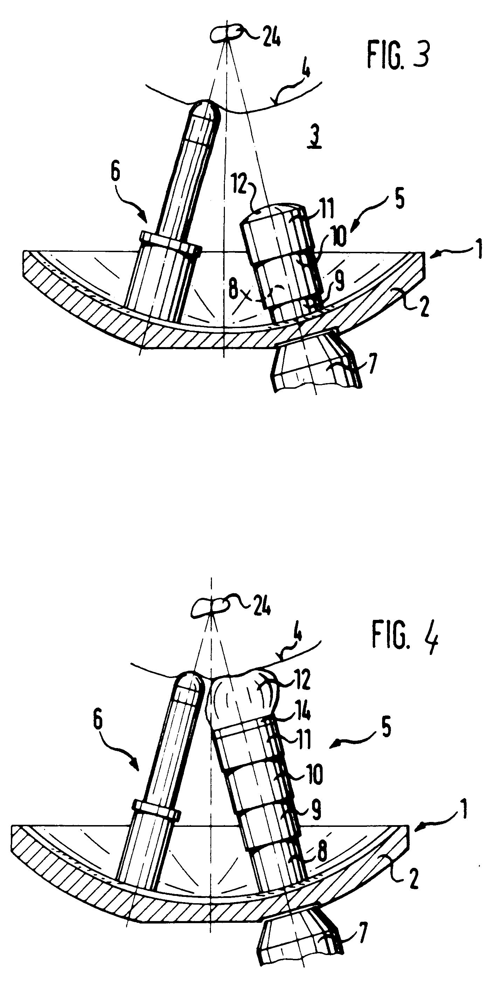

Durch Einleiten von Gas in den Röntgentubus 8 kann dessen Länge stufenlos von der in Figur 2 dargestellten ersten Endstellung bis in die in Figur 4 dargestellte zweite Endstellung, bei der das den Röntgentubus abschließende Ballonende an der Membran 4 anliegt, verstellt werden. Durch weitere Zufuhr von Gas wird der z.B. aus Latex bestehende an der Innenwand des Röntgentubus 8 anliegende und radial durch die Rohrabschnitte 9,10,11 abgestützte röntgenfähige Ballon 12 vollständig entfaltet, wodurch das flüssige Koppelmedium vollständig verdrängt wird und der Ballon an der Membran 4 der Kalotte anliegt. Durch Evakuieren des Ballons 12 über den Kanal 18 und die Leitung 20 kann der Röntgentubus 8 auf seine geringste Länge reduziert werden.

Die Figuren 2 bis 4 zeigen unterschiedliche Betriebsstellungen der Ultraschallortungseinrichtung 6 und der Röntgenortungseinrichtung 5. Bei der in Figur 2 dargestellten Stellung sind sowohl die Ultraschallortungseinrichtung 4 als auch der Röntgentubus 8 der Röntgenortungseinrichtung 5 in eine membranferne Stellung gebracht, in der keine oder die geringstmögliche Abschattung des Schallfeldes gegeben ist. In dieser Stellung ist eine in-situ-Röntgen- und Ultraschallortung sowie eine besonders effiziente Stoßwellenapplikation möglich.

Figur 3 zeigt die Ultraschallortungseinrichtung 6 in der membrannahen Stellung, wobei die Länge des Röntgentubus 8 der Röntgenortungseinrichtung 5 etwa auf die halbe Länge reduziert ist. In dieser Stellung gibt sich für die Ultraschallortungseinrichtung 6 eine optimale und für die Röntgenortungseinrichtung 5 eine gegenüber der in Figur 2 dargestellten Stellung verbesserte Bildqualität, während die Effizienz der Stoßwellenapplikation aufgrund der durch die Ortungseinrichtungen 5 und 6 hervorgerufenen Abschattung des Schallfeldes nur geringfügig verschlechtert und für eine wirkungsvolle Stoßwellenbehandlung noch vertretbar ist.

Figur 4 zeigt eine Stellung, bei der sowohl die Ultraschallortungseinrichtung 6 als auch die Röntgenortungseinrichtung 5 an der Membran 4 anliegen. In dieser Stellung ist eine optimale Bilddarstellung des innerhalb des Körpers der zu behandelnden Person liegenden Konkrements 24 oder Gewebes gegeben, wobei in dieser Position durch die starke Abschattung des Schallfeldes durch die Ortungseinrichtungen 5 und 6 eine effiziente Stoßwellenbehandlung nicht mehr möglich ist.

Durch den stufenlos längenverstellbaren Röntgentubus 8 kann die Abschattung des Schallfeldes zu Zwecken der Therapie minimiert werden und zum Zwecke der Ortung die Auflösung der Röntgenortungseinrichtung 5 optimiert werden. Je nach Anwendungsfall kann der Therapeut zwischen den jeweiligen Optima frei wählen und so durch die Ortung des Konkrements 24 bzw. Gewebes einerseits und Zerstörung des Konkrements 24 mittels Stoßwellen andererseits die Behandlung optimieren.

## Patentansprüche

1. Vorrichtung zur Stoßwellenbehandlung mit einer an einen Patienten anlegbaren, durch eine Membran (4) abgeschlossenen und mit einem Ankopplungsmedium (3) gefüllten Vorlaufstrecke eines Stoßwellenwandlers (1) und mit einer Röntgeneinrichtung (5) zur Ortung des durch die Stoßwellen zu zerstörenden Konkrementes oder Gewebes, deren Röntgenstrahler (7) einen innerhalb der Vorlaufstrecke angeordneten, mit einem für Röntgenstrahlen verlustfreien Medium füllbaren und gegenüber dem Ankoppelmedium (3) abgedichteten Tubus (8) aufweist, der an seinem freien Ende durch einen aufblasbaren Ballon (12) abgeschlossen ist, wobei der Tubus (8) innerhalb der Vorlaufstrecke in Richtung seiner Längsachse längenverstellbar ausgebildet ist.

2. Vorrichtung nach Anspruch 1, dadurch gekennzeichnet, daß der Tubus (8) teleskopartig ausgebildet ist.

3. Vorrichtung nach Anspruch 1 oder 2, dadurch gekennzeichnet, daß die Längenverstellung des Tubus (8) durch Anlegen eines Über- und Unterdruckes steuerbar ist.

4. Vorrichtung nach Anspruch 1, dadurch gekennzeichnet, daß der Ballon (12) innerhalb des Tubus (8) längs der Tubuswand verläuft.

5. Vorrichtung nach Anspruch 1 oder 4, dadurch gekennzeichnet, daß der Ballon (12) am membranfernen Ende des Tubus (8) mittels eines den Ballon (12) gasdicht an die Tubuswand anlegenden ersten Klemmringes (13) befestigt ist.

6. Vorrichtung nach Anspruch 1, 4 oder 5, dadurch gekennzeichnet, daß der Ballon (12) nahe dem freien Ende des Tubus (8) mittels eines den Ballon (12) an die Tubuswand anlegenden zweiten Klemmringes (14) befestigt ist.

## Claims

1. A device for shock wave treatment with a forward stage of a shock wave transducer (1) able to be applied to a patient, closed off by a membrane (4) and filled with a coupling medium (3), and with an X-ray device (5) for locating the concretion or tissue which is to be destroyed by the shock waves, the X-ray emitter (7) of which has a tube (8) which is arranged within the forward stage, is able to be filled with a loss-free medium for X-rays and is sealed off with respect to the coupling medium (3), which tube (8) is closed off at its free end by an inflatable balloon (12), in which the tube (8) is constructed so as to be adjustable in length within the forward stage in the direction of its longitudinal axis.

2. A device according to claim 1, characterised in that the tube (8) is constructed in the manner of a telescope.

3. A device according to Claim 1 or 2, characterised in that the longitudinal adjustment of the tube (8) is able to be controlled by the application of an excess pressure and an under-pressure.

4. A device according to Claim 1, characterised in that the balloon (12) runs inside the tube (8) along the tube wall.

5. A device according to Claim 1 or 4, characterised in that the balloon (12) is secured at the end of the tube (8) away from the membrane by means of a first clamping ring (13) placing the balloon (12) in a gas-tight manner against the tube wall.

6. A device according to claim 1, 4 or 5, characterised in that the balloon (12) is secured close to the free end of the tube (8) by means of a second clamping ring (14) placing the balloon (12) against the tube wall.

## Revendications

1. Dispositif de traitement par ondes de choc comportant une section amont d'un transducteur à ondes de choc (1), qui peut être appliquée contre un patient, et est fermée par une membrane (4) et remplie par un milieu de couplage (3), et un dispositif à rayons X (5) pour localiser la concrétion ou le tissu devant être détruit par les ondes de choc et dont l'émetteur de rayons X (7) possède un tube (8) qui est disposé à l'intérieur de la section amont, peut être rempli par un milieu sans pertes pour les rayons X et qui est étanchéifié par rapport au milieu de couplage (3) et dont l'extrémité libre est fermée par un ballon gonflable (12), le tube (8) étant réalisé, à l'intérieur de la section amont, de manière à être réglable en longueur dans la direction de son axe longitudinal.

2. Dispositif selon la revendication 1, caractérisé en ce que le tube (8) présente un agencement télescopique.

3. Dispositif selon la revendication 1 ou 2, caractérisé en ce que le réglage en longueur du tube (8) est commandable au moyen de l'application d'une surpression et d'une dépression.

4. Dispositif selon la revendication 1, caractérisé en ce que le ballon (12) s'étend à l'intérieur du tube (8), le long de la paroi de ce dernier.

5. Dispositif selon la revendication 1 ou 4, caractérisé en ce que le ballon (12) est fixé, au niveau de l'extrémité du tube (8), éloignée de la membrane, au moyen d'une première bague de serrage (13), qui applique d'une manière au gaz étanche le ballon (12) contre la paroi du tube.

6. Dispositif selon la revendication 1, 4 ou 5, caractérisé en ce que le ballon (12) est fixé, à proximité de l'extrémité libre du tube (8), au moyen d'une seconde bague de serrage (14), qui applique le ballon (12) contre la paroi du tube.
